# EUROPEAN PATENT APPLICATION

(11) **EP 1 323 426 A1**
(43) Date of publication of application: **02.07.2003**
(21) Application number: 01970304.0
(22) Date of filing: 28.09.2001
(51) Int. Cl.: A61K 35/78, A61K 9/08, A61K 47/46, A61K 31/125, A61K 31/045, A61K 31/122, A61K 31/015, A61P 27/02

(54) **EYE DROPS**

(30) Priority: 03.10.2000 JP 2000303485
(71) Applicant: Senju Pharmaceutical Co., Ltd., Osaka-shi, Osaka 541-0046 (JP)
(72) Inventor: NAKAYAMA, Hisayuki, Nishinomiya-shi, Hyogo 622-0826 (JP); AKI, Hiroshi, Kobe-shi, Hyogo 657-0034 (JP); NEMOTO, Fukiko, Kobe-shi, Hyogo 651-2109 (JP)
(74) Representative: von Kreisler, Alek, Dipl.-Chem.
(86) International application number: JP0108605
(87) International publication number: WO02028404

(57) **Abstract**

Eye drops are disclosed to which ginger perfume is added as a refreshing agent.

## Description

### TECHNICAL FIELD

The present invention relates to eye drops containing ginger perfume.

### BACKGROUND OF THE INVENTION

Eye drops are widely used for treatment of allergy and inflammation of the cornea and conjunctiva, infection of the cornea and conjunctiva, glaucoma, cataract and the like and xerosis corneae and the like, prevention of corneal disorders while wearing contact lenses, and treatment of eye fatigue of various degree including asthenopia, and a variety of ophthalmologic diseases and symptoms such as irritation, foreign-body sensation and the like. Among them, such eye drops are widely available in the market that contain one or more ingredients which serve, upon instillation, to provide refreshing stimulation to the eye (hereinafter referred to as "refreshing agents") for refreshment, and are intended mainly for uncomfortable subjective symptoms like comparatively mild eye fatigue or itching or to drowsiness.

However, only a very limited number of compounds are known as refreshing agents for eye drops, such as menthol and borneol. Considering the diversity of recent consumers' taste, there must be potential needs, with respect to eye drops which provide refreshing feeling, also for product groups employing a wider variety of refreshing agents than before.

Against the above background, it is the purpose of the present invention to provide eye drops containing a refreshing agent different from those which have been known.

Apart from nutritional ingredients such as starch, fiber, minerals, and trace amount of protein and fat, ginger rhizome contains a variety of peculiar ingredients which provide basis for its uses for seasonings, cosmetics and as crude drugs. Those include, for example, (6)-gingerol, (8)-gingerol, (10)-gingerol, zingerone, (6)-shogaol, (8)-shogaol, (10)-shogaol, zingiberene, (6)-paradol, (8)-paradol, (10)-paradol, zingiberol, (4)-gingediol, (6)-gingediol, (8)-gingediol, (10)-gingediol, (6)-methylgingediol, (4)-gingediacetate, (6)-gingediacetate, hexahydrocurcumin, curcumin, bisabolene, sesquiphellandrene, pinene, myrcene, farnesene, geraniol, limonene, neral and the like.

Ginger rhizome is used as an aromatic stomachic, as a crude drug for enhancing metabolism and preventing increase in blood cholesterol, and as a crude drug for alleviating chest pain, abdominal pain, backache, diarrhea and the like. It is also used for promoting sweating against a cold. However, its use as a refreshing agent for eye drops has not been known.

### DISCLOSURE OF THE INVENTION

In the process of investigation for a novel refreshing agent for eye drops, the present inventors have found that ingredients extracted from ginger rhizome can be used for the purpose. The present invention has been completed as a result of studies based on the findings.

Thus, the present invention provides eye drops to which ginger perfume is added as a refreshing agent. Wherein the concentration of ginger perfume can be selected as desired, but is usually 0.00005 to 1.0 w/v%, preferably 0.0001 to 0.5 w/v%, and more preferably 0.001 to 0.3 w/v%.

Addition of ginger perfume to eye drops may be made by addition of ginger perfume or by addition of the ingredients extracted from ginger rhizome. Therefore, the present invention also provides eye drops to which the ingredients extracted from ginger rhizome are added as a refreshing agent.

### BEST MODE FOR CARRYING OUT THE INVENTION

In the present invention, "eye drops" may be eye drops in various dosage forms which are used by instilling drops to the eye. As aqueous eye drops, there are included eye drops in which water is used as a base and various essential ingredients (for example, active ingredients, isotonizer, buffer, preservative and the like) are dissolved or dispersed (suspended, emulsified and the like) in it in accordance with the purpose of the eye drops.

In the present invention, the phrase "ingredients extracted from ginger rhizome" means ingredients which can be obtained by extraction from ginger rhizome by various methods, regardless of the means for extraction. Ginger rhizome, either raw or dried, may be used. In order to use those ingredients in eye drops of the present invention, those peculiar ingredients contained in ginger rhizome may be added to the eye drops which were collected by extraction from ginger rhizome with water (warm water or hot water), extraction with organic solvents such as acetone, ethanol, chloroform, benzene and diethyl ether, extraction with water and an organic solvent, or by distillation from hot water, steam distillation, with carbon dioxide and the like. It is also possible to use one or more synthetic products of those ingredients extractable from ginger rhizome, for example, zingerone, gingerol, curcumin, pinene, myrcene, farnesene, geraniol, limonene and the like.

In the present invention, such ginger perfume may be used that contains, usually in a liquid medium, extracts of ginger rhizome or its main ingredients, in particular, aromatic ingredients. A ginger perfume employed may be a product which is commercially available. Examples of commercially available ginger perfumes include Ginger Flavor manufactured by OGAWA & CO., LTD., Ginger Oil manufactured by TAKASAGO INTERNATIONAL CORPORATION, and the like.

There is no definite range within which the amount of ginger perfume added to eye drops should fall, but such a range, in general, may be from 0.00005 to 1.0 w/v%, preferably from 0.0001 to 0.5 w/v%, and more preferably from 0.001 to 0.3 w/v%, in accordance with intended degree of refreshing feeling to be provided, from slightly felt refreshing feeling to the strong refreshing feeling.

As to other ingredients of eye drops than those extracted from ginger rhizome, it is possible, like conventional eye drops, to add various basic ingredients such as isotonizers, buffers, pH adjusting agents, preservatives and surfactants, and various active ingredients such as vitamins, vasoconstrictor, antihistamic agents, anti-inflammatory agents, antiallergic agents and antimicrobials as desired and in accordance with respective purposes. Thus, for example, ingredients extracted from ginger rhizome may be added in place of such refreshing agents as 1-menthol and borneol, in various eye drops which were formulated using those refreshing agents. Further, a refreshing agent such as a monoterpenoid may be added to the eye drops of the present invention, together with ginger perfume. Eye drops having deeper refreshing feeling can be obtained by addition of a refreshing agent such as a monoterpenoid. Examples of monoterpenoids include menthol, camphor, borneol, geraniol, menthone, cineol, limonene, eugenol, citral, pinene, linalol, fenchyl alcohol, ionone, safranal, terpinene and the like. Among these, menthol, camphor, borneol, geraniol, menthone, cineol and limonene are used particularly preferably. These can be used alone or two or more of them can be used in combination as desired. The concentration of monoterpenoids is usually from 0.0005 to 0.1 w/v% and preferably from 0.001 to 0.06 w/v%, though it depends on the amount of ginger perfume added.

Further, the eye drops of the present invention may be instilled while wearing contact lenses of various types such as non-oxygen gas permeable hard contact lenses, oxygen gas permeable hard contact lenses and soft contact lenses, and, in particular, advantageously instilled while wearing soft contact lenses.

### [Examples]

The present invention will be illustrated below in further detail with reference to Reference Examples and Examples. However, it is not intended that the present invention be limited to those Examples.

### [Reference Example 1] Extraction of ingredients from ginger rhizome

Tow hundred g of raw ginger rhizome is minced and combined with 300 ml of acetone, and extraction is carried out for about 2 hours by refluxing by heating while stirring. After extraction, the rhizome and other unnecessary portions are removed by filtration, and the solvent is evaporated under reduced pressure to obtain crude extract. Water and chloroform are added to the crude extract and mixed by shaking, chloroform phase is collected, and ginger rhizome extract is obtained by evaporating the solvent under reduced pressure.

### [Reference Example 2] Extraction of ingredients from ginger rhizome

One hundred g of dried ginger rhizome is minced and combined with 300 ml of purified water, and extraction is carried out for about 2 hours by refluxing by heating while stirring. After extraction and before cooling, the rhizome and other unnecessary portions are removed by filtration, and the solvent is evaporated under reduced pressure to obtain crude extract. Water and chloroform are added to the crude extract and mixed by shaking, chloroform phase is collected, and ginger rhizome extract is obtained by evaporating the solvent under reduced pressure.

### [Reference Example 3] Extraction of ingredients from ginger rhizome

Three hundred g of raw ginger rhizome is minced, and placed in a round-bottom flask (A) together with 300 ml of purified water. While boiling the mixture, water vapor from boiling purified water contained in another round-bottom flask (B) is introduced for about 1 hour, and the distilled water vapor from the round-bottom flask (A) is cooled through a Liebig condenser and collected. Chloroform is added to the extract obtained and mixed by shaking, chloroform phase is collected, and the extract of ginger rhizome is obtained by evaporating the solvent under reduced pressure.

### [Reference Example 4] Extraction of ingredients from ginger rhizome

Two hundred g of the dried ginger rhizome is minced, and placed in the extraction chamber of a supercritical carbon dioxide extraction equipment, and the equipment is run. Supercritical carbon dioxide is introduced in the extraction chamber of the equipment. The supercritical carbon dioxide flowing out after being contacted with the minced dried ginger rhizome is then introduced into a separation chamber, which are under normal pressure. Carbon dioxide is separated from the extracted ingredients in the separation chamber and evaporates, giving ginger rhizome extract as an oil left in the separation chamber.

### <Test of refreshing feeling >

When eye drops containing conventional refreshing agents such as 1-menthol and borneol is instilled in the eyes of a rabbit, the degree of feeling of stimulation by the refreshing agent correlates with the length of time from immediately after instillation to restoration of the baseline condition, in which the eyes are persistently open, and with the number of times of blinking during the period. Based on this, the refreshing feeling felt upon instillation of eye drops containing ginger rhizome-extracted ingredients was compared with that caused by 1-menthol, which is a typical refreshing agent for eye drops, using as the index the number of times of blinking in a rabbit immediately after instillation. Commercially available ginger perfume was used as ginger rhizome-extracted ingredients.

### (Methods)

Albino rabbits were divided into groups, six rabbits each. The eye drops of the following formulas (TP-26, TP-27 and TP-30) and saline were instilled once (2 drops) in one or the other of the both eyes of the rabbits in each group, after which the length of time from immediately after instillation to restoration of the baseline condition, in which the eyes are persistently open, was measured, and the number of times of blinking during the period was counted.

**Table 1**

| | TP-26 | TP-27 | TP-30 |
|---|---|---|---|
| Sodium chloride | 0.9 g | 0.9 g | 0.9 g |
| 1-Menthol | 0.002 g | 0.002 g | - |
| Ginger perfume* | 0.2 g | - | 0.2g |
| HCO60** | 0.3 g | 0.3g | 0.3g |
| Sodium hydroxide | q.s. | q.s. | q.s. |
| Sterilized purified water | q.s. | q.s. | q.s. |
| Total amount (ml) | 100 | 100 | 100 |
| pH | 5.5 | 5.5 | 5.5 |

| | | | |
|---|---|---|---|
| * Ginger perfume: Ginger Flavor R70549 (manufactured by OGAWA & CO., LTD.) | | | |
| ** Polyoxyethylenehydrogenated castor oil | | | |

### (Result)

The following table shows the length of time required for restoration of the condition in which the eyes are consistently open and the number of times of blinking during the period, as mean values of each group.

**Table 2**

| Formulas | Measurement items | Results |
|---|---|---|
| TP-26 | Time for restoration* | 226.7 seconds |
| | Number of times of blinking | 7.2 times |
| TP-27 | Time for restoration* | 143.9 seconds |
| | Number of times of blinking | 3.3 times |
| TP-30 | Time for restoration* | 473.9 seconds |
| | Number of times of blinking | 15.6 times |
| Saline | Time for restoration* | 25.3 seconds |
| | Number of times of blinking | 1.3 times |

| | | |
|---|---|---|
| * Length of time from immediately after instillation to restoration of the baseline condition, in which the eyes are persistently open. | | |

As shown in Table 2, with formula TP-30, remarkably prolonged time for restoration (about 3-fold) and increased number of times of blinking (about 5-fold) were noted compared with those with formula TP-27, which contained 1-menthol as a refreshing agent. This means that ginger perfume at the above-mentioned concentration is several-times more potent than 0.002 w/v% 1-menthol with regard to refreshing feeling. Further, with formula TP-26 containing both ginger perfume and 1-menthol, both of the time for restoration and the number of times of blinking were about one half of those with TP-30. This seems to be due to the anesthetic effect of 1-menthol, which might have weaken the refreshing stimulation by ginger perfume. Further, no irritation to tissues was observed in any of the rabbits in gross visual observation of the cornea, conjunctiva and nictitating membrane.

### [Example 1]

| | |
|---|---|
| Tetrahydrozoline hydrochloride | 0.05 g |
| Chlorpheniramine maleate | 0.03 g |
| Potassium L-aspartate | 1 g |
| Sodium chloride. | 0.5 g |
| Sodium L-glutamate | 0.2 g |
| Benzalkonium chloride | 0.005 g |
| Chlorobutanol | 0.2 g |
| 1-Menthol | 0.01 g |
| Ginger rhizome extract | 0.2 g |
| Polyoxyethylenehydrogenated castor oil 60 | 0.3 g |
| Sodium hydroxide | q.s. |
| Purified water | q.s. |
| Total amount | 100 ml (pH = 5.5) |

Chlorobutanol, tetrahydrozoline hydrochloride, chlorpheniramine maleate, potassium L-aspartate, sodium chloride, sodium L-glutamate and benzalkonium chloride were sequentially dissolved in about 70 ml of purified water, and to the solution were added 1-menthol and the ginger rhizome extract (the acetone extract in Reference Example 1) dispersed with polyoxyethylenehydrogenated castor oil 60, and, after pH was adjusted to 5.5 by sodium hydroxide, purified water was added to make the total volume of 100 ml. This was sterilized by filtration, and filled in 15-ml containers of polyethylene terephthalate.

### [Example 2]

| | |
|---|---|
| Chlorpheniramine maleate | 0.03 g |
| Dipotassium glycyrrhizinate | 0.25 g |
| Sodium chloride | 0.5 g |
| Sodium L-glutamate | 0.2 g |
| Benzalkonium chloride | 0.005 g |
| Chlorobutanol | 0.2 g |
| 1-Menthol | 0.01 g |
| Ginger rhizome extract | 0.2 g |
| Polyoxyethylenehydrogenated castor oil 60 | 0.3 g |
| Sodium hydroxide | q.s. |
| Purified water | q.s. |
| Total amount | 100 ml (pH = 5.5) |

Chlorobutanol, chlorpheniramine maleate, dipotassium glycyrrhizinate, sodium chloride, sodium L-glutamate and benzalkonium chloride were sequentially dissolved in about 70 ml of purified water, and to the solution were added 1-menthol and the ginger rhizome extract (the hot water extract in Reference Example 2) dispersed with polyoxyethylenehydrogenated castor oil 60, and, after pH was adjusted to 5.5 by sodium hydroxide, purified water was added to make the total volume of 100 ml. This was sterilized by filtration, and filled in 15-ml containers of polyethylene terephthalate.

### [Example 3]

| | |
|---|---|
| Tetrahydrozoline hydrochloride | 0.05 g |
| Chlorpheniramine maleate | 0.03 g |
| Potassium L-aspartate | 1 g |
| Sodium chloride | 0.5 g |
| Sodium L-glutamate | 0.2 g |
| Benzalkonium chloride | 0.005 g |
| Chlorobutanol | 0.2 g |
| 1-Menthol | 0.03 g |
| dl-Camphor | 0.01 g |
| Borneol | 0.01 g |
| Extract of ginger rhizome | 0.01 g |
| Polyoxyethylene hydrogenated castor oil 60 | 0.3 g |
| Sodium hydroxide | q.s. |
| Purified water | q.s. |
| Total amount | 100 ml (pH = 5.5) |

Chlorobutanol, tetrahydrozoline hydrochloride, chlorpheniramine maleate, potassium L-aspartate, sodium chloride, sodium L-glutamate and benzalkonium chloride were sequentially dissolved in about 70 ml of purified water, and to this were added 1-menthol, dl-camphor, borneol and the ginger rhizome extract (the acetone extract in Reference Example 1) dispersed with polyoxyethylenehydrogenated castor oil 60, and, after pH was adjusted to 5.5 by sodium hydroxide, purified water was added to make the total volume of 100 ml. This was sterilized by filtration, and filled in 15-ml containers of polyethylene terephthalate.

### [Example 4]

| | |
|---|---|
| Tetrahydrozoline hydrochloride | 0.05 g |
| Chlorpheniramine maleate | 0.03 g |
| Potassium L-aspartate | 1 g |
| Sodium chloride | 0.5 g |
| Sodium L-glutamate | 0.2 g |
| Benzalkonium chloride | 0.005 g |
| Chlorobutanol | 0.2 g |
| 1-Menthol | 0.01 g |
| Ginger rhizome extract | 0.05 g |
| Polysorbate 80 | 0.2 g |
| Sodium hydroxide | q.s. |
| Purified water | q.s. |
| Total amount | 100 ml (pH = 5.5) |

Chlorobutanol, tetrahydrozoline hydrochloride, chlorpheniramine maleate, potassium L-aspartate, sodium chloride, sodium L-glutamate and benzalkonium chloride were sequentially dissolved in about 70 ml of purified water, and to this were added 1-menthol and the ginger rhizome extract (the hot water extract in Reference Example 2) dispersed with polysorbate 80, and, after pH was adjusted to 5.5 by sodium hydroxide, purified water was added to make the total volume of 100 ml. This was sterilized by filtration, and filled in 15-ml containers of polyethylene terephthalate.

### [Example 5]

| | |
|---|---|
| Tetrahydrozoline hydrochloride | 0.05 g |
| Chlorpheniramine maleate | 0.03 g |
| Aminoethylsulfonic acid | 1.0 g |
| Sodium chloride | 0.5 g |
| Sodium L-glutamate | 0.2 g |
| Benzalkonium chloride | 0.005 g |
| Chlorobutanol | 0.2 g |
| 1-Menthol | 0.01 g |
| Ginger Flavor R70549* | 0.2 g |
| Polyoxyethylenehydrogenated castor oil 60 | 0.3 g |
| Sodium hydroxide | q.s. |
| Purified water | q.s. |
| Total amount | 100 ml (pH = 5.5) |

| | |
|---|---|
| * Ginger Flavor (extract by steam distillation) manufactured by OGAWA & CO., LTD. | |

Chlorobutanol, tetrahydrozoline hydrochloride, chlorpheniramine maleate, aminoethylsulfonic acid, sodium chloride, sodium L-glutamate and benzalkonium chloride were sequentially dissolved in about 70 ml of purified water, and to this were added 1-menthol and Ginger Flavor dispersed with polyoxyethylenehydrogenated castor oil 60, and, after pH was adjusted to 5.5 by sodium hydroxide, purified water was added to make the total volume of 100 ml. This was sterilized by filtration, and filled in 15-ml containers of polyethylene terephthalate.

### [Example 6]

| | |
|---|---|
| Tetrahydrozoline hydrochloride | 0.05 g |
| Chlorpheniramine maleate | 0.03 g |
| Aminoethylsulfonic acid | 1.0 g |
| Sodium chloride | 0.5 g |
| Sodium L-glutamate | 0.2 g |
| Benzalkonium chloride | 0.005 g |
| Chlorobutanol | 0.2 g |
| 1-Menthol | 0.04 g |
| Ginger Oil T1* | 0.02 g |
| Polyoxyethylenehydrogenated castor oil 60 | 0.3 g |
| Sodium hydroxide | q.s. |
| Purified water | q.s. |
| Total amount | 100 ml (pH = 5.5) |

| | |
|---|---|
| * Ginger Perfume manufactured by TAKASAGO INTERNATIONAL CORPORATION. | |

Chlorobutanol, tetrahydrozoline hydrochloride, chlorpheniramine maleate, aminoethylsulfonic acid, sodium chloride, sodium L-glutamate and benzalkonium chloride were sequentially dissolved in about 70 ml of purified water, and to this were added 1-menthol and Ginger Oil T1 dispersed with polyoxyethylenehydrogenated castor oil 60, and, after pH was adjusted to 5.5 by sodium hydroxide, purified water was added to make the total volume of 100 ml. This was sterilized by filtration, and filled in 15-ml containers of polyethylene terephthalate.

### [Example 7]

| | |
|---|---|
| Chlorpheniramine maleate | 0.03 g |
| Sodium chloride | 0.5 g |
| Benzalkonium chloride | 0.005 g |
| Chlorobutanol | 0.2 g |
| 1-Menthol | 0.04 g |
| Zingerone* | 0.0005 g |
| Polyoxyethylenehydrogenated castor oil 60 | 0.3 g |
| Sodium hydroxide | q.s. |
| Purified water | q.s. |
| Total amount | 100 ml (pH = 5.5) |

| | |
|---|---|
| * manufactured by ALDRICH CHEMICAL COMPANY | |

Chlorobutanol, chlorpheniramine maleate, sodium chloride and benzalkonium chloride were dissolved in about 70 ml of purified water, and to this were added 1-menthol and zingerone dispersed with polyoxyethylenehydrogenated castor oil 60, and, after pH was adjusted to 5.5 by sodium hydroxide, purified water was added to make the total volume of 100 ml. This was sterilized by filtration, and filled in 15-ml containers of polyethylene terephthalate.

### [Example 8]

| | |
|---|---|
| Naphazoline hydrochloride | 0.003 g |
| Neostigmine methylsulfate | 0.005 g |
| Allantoin | 0.1 g |
| Chlorpheniramine maleate | 0.03 g |
| Sodium chondroitin sulfate | 0.1 g |
| Boric acid | 1 g |
| Benzalkonium chloride | 0.005 g |
| Chlorobutanol | 0.2 g |
| Ginger rhizome extract | 0.001 g |
| Polyoxyethylenehydrogenated castor oil 60 | 0.3 g |
| Hydrochloric acid | q.s. |
| Sodium hydroxide | q.s. |
| Purified water | q.s. |
| Total amount | 100ml (pH = 5.5) |

Chlorobutanol, naphazoline hydrochloride, neostigmine methylsulfate, allantoin, chlorpheniramine maleate, sodium chondroitin sulfate, boric acid and benzalkonium chloride were sequentially dissolved in about 70 ml of purified water, and to this was added the ginger rhizome extract (the extract by carbon dioxide in Reference Example 4) dispersed with polyoxyethylenehydrogenated castor oil 60, and, after pH was adjusted to 5.5 by hydrochloric acid or sodium hydroxide, purified water was added to make the total volume of 100 ml. This was sterilized by filtration, and filled in 15-ml containers of polyethylene terephthalate.

### [Example 9]

| | |
|---|---|
| Neostigmine methylsulfate | 0.005 g |
| Allantoin | 0.1 g |
| Chlorpheniramine maleate | 0.03 g |
| Panthenol | 0.05 g |
| Potassium L-aspartate | 1 g |
| Boric acid | 1 g |
| Benzalkonium chloride | 0.005 g |
| Chlorobutanol | 0.2 g |
| Ginger India PFICO₂* | 0.005 g |
| Polysorbate 80 | 0.3 g |
| Hydrochloric acid | q.s. |
| Sodium hydroxide | q.s. |
| Purified water | q.s. |
| Total amount | 100 ml (pH = 5.5) |

| | |
|---|---|
| * Ginger Perfume (extract by carbon dioxide) sold by MORIMURA BROS., INC. | |

Chlorobutanol, neostigmine methylsulfate, allantoin, chlorpheniramine maleate, panthenol, potassium L-aspartate, boric acid and benzalkonium chloride were sequentially dissolved in about 70 ml of purified water, and to this was added Ginger India PFICO₂ dispersed with polysorbate 80, and, after pH was adjusted to 5.5 by hydrochloric acid or sodium hydroxide, purified water was added to make the total volume of 100 ml. This was sterilized by filtration, and filled in 15-ml containers of polyethylene terephthalate.

### [Example 10]

| | |
|---|---|
| Naphazoline hydrochloride | 0.003 g |
| Dipotassium glycyrrhizinate | 0.1 g |
| Diphenhydramine hydrochloride | 0.05 g |
| d-α-Tocopherol acetate | 0.05 g |
| Aminoethylsulfonic acid | 1 g |
| Boric acid | 1 g |
| Benzalkonium chloride | 0.005 g |
| Chlorobutanol | 0.2 g |
| Ginger rhizome extract | 0.1 g |
| Polysorbate 80 | 0.3 g |
| Hydrochloric acid | q.s. |
| Sodium hydroxide | q.s. |
| Purified water | q.s. |
| Total amount | 1.00ml (pH = 5.5) |

Chlorobutanol, naphazoline hydrochloride, dipotassium glycyrrhizinate, diphenhydramine hydrochloride, aminoethylsulfonic acid and boric acid were sequentially dissolved in about 70 ml of purified water, and to this were added d-α-tocopherol acetate dispersed with polyoxyethylenehydrogenated castor oil 60 and the ginger rhizome extract (the extract by steam distillation in Reference Example 3) dispersed with polysorbate 80, and, after pH was adjusted to 5.5 by hydrochloric acid or sodium hydroxide, purified water was added to make the total volume of 100 ml. This was sterilized by filtration, and filled in 15-ml containers of polyethylene terephthalate.

### [Example 11]

| | |
|---|---|
| Sodium chloride | 0.55 g |
| Potassium chloride | 0.15 g |
| Boric acid | 0.5 g |
| Borax | 0.05 g |
| Benzalkonium chloride | 0.005 g |
| Ginger Essential Oil* | 0.02 g |
| Polysorbate 80 | 0.3 g |
| Purified water | q.s. |
| Total amount | 100 ml (pH = 7.3) |

| | |
|---|---|
| * Ginger Perfume (extract by steam distillation) sold by TREE OF LIFE CO., LTD. | |

Sodium chloride, potassium chloride, boric acid, borax and benzalkonium chloride were sequentially dissolved in about 70 ml of purified water, and to this was added Ginger Essential Oil dispersed with polysorbate 80, and purified water was added to make the total volume of 100 ml. This was sterilized by filtration, and filled in 15-ml containers of polyethylene terephthalate.

### [Example 12]

| | |
|---|---|
| Ginger Flavor R70544* | 0.02 g |
| 1-Menthol | 0.0025 g |
| Sodium chloride | 0.9 g |
| Polyoxyethylenehydrogenated castor oil 60 | 0.2 g |
| Hydrochloric acid | q.s. |
| Sodium hydroxide | q.s. |
| Purified water | q.s. |
| Total amount | 100 ml (pH = 5.5) |

| | |
|---|---|
| * Ginger Flavor (extract by steam distillation) manufactured by OGAWA & CO., LTD. | |

Sodium chloride was dissolved in about 70 ml of purified water, and to this were added 1-menthol and Ginger Flavor R70544 dispersed with polyoxyethylene hydrogenated castor oil 60, and, after pH was adjusted to 5.5 by hydrochloric acid or sodium hydroxide, purified water was added to make the total volume of 100 ml. This was sterilized by filtration, and filled in 15-ml containers of polyethylene terephthalate.

### INDUSTRIAL APPLICABILITY

The present invention can provide eye drops containing a refreshing agent which is a totally different type from conventional ones.

## Claims

1. Eye drops to which ginger perfume is added as a refreshing agent.

2. The eye drops according to claim 1, wherein the concentration of the ginger perfume is from 0.00005 to 1.0 w/v%.

3. The eye drops according to claim 1 or 2, wherein the ginger perfume is an extract of ginger rhizome.

4. The eye drops according to claim 3, wherein the extract of ginger rhizome is an extract with an organic solvent.

5. The eye drops according to claim 3, wherein the extract of ginger rhizome is an extract with hot water.

6. The eye drops according to claim 3, wherein the extract of ginger rhizome is an extract by steam distillation.

7. The eye drops according to claim 3, wherein the extract of ginger rhizome is an extract with carbon dioxide.

8. The eye drops according to one of claims 3 to 7, wherein the concentration of the extract of ginger rhizome is from 0.001 to 0.3 w/v%.

9. The eye drops according to one of claims 1 to 8, to which a monoterpenoid is further added.

10. The eye drops according to claim 9, wherein the monoterpenoid is at least one selected from a group consisting of menthol, camphor, borneol, geraniol, menthone, cineol and limonene.

11. The eye drops according to claim 9 or 10, wherein the concentration of the monoterpenoid is from 0.0005 to 0.1 w/v%.

12. The eye drops according to one of claims 1 to 11, wherein the eye drops are eye drops for use while wearing a contact lens.

13. A method for production of the eye drops according to one of claims 1 to 12, wherein the method comprises adding ginger perfume to eye drops.
